# EUROPEAN PATENT SPECIFICATION

(11) **EP 1 992 313 B1**
(45) Date of publication and mention of the grant of the patent: **14.03.2012**
(21) Application number: 08251684.0
(22) Date of filing: 13.05.2008
(51) Int. Cl.: A61F 5/00

(54) **Gastric band with engagement member**
Magenband mit Einhakglied
Bande gastrique dotée d'un élément d'engagement

(30) Priority: 14.05.2007 US 798501
(43) Date of publication of application: 19.11.2008
(73) Proprietor: Ethicon Endo-Surgery, Inc., Cincinnati, OH 45242 (US)
(72) Inventor: Weaner, Lauren S., Beavercreek, OH 45434 (US); Wiley, Jeffrey P., Milford, OH 45150 (US); Jambor, Kristin L., Cincinnati, OH 45208 (US); Widenhouse, Christopher W., Clarksville, OH 45113 (US); Swindon, Patrick J., Cincinnati, OH 45208 (US)
(74) Representative: Tunstall, Christopher Stephen

(56) References cited:
- WO-A-01/85071
- WO-A-2005/072195
- WO-A-2007/080334
- WO-A-2008/109300
- US-A1- 2005 119 674
- US-A1- 2005 228 415
- US-A1- 2007 016 229

## Description

### 1. Field of the Invention

The invention relates to a gastric band and related accessories. More particularly, the invention relates to a gastric band including an engagement member enhancing manipulation thereof during application and removal of a gastric band about a stomach.

### 2. Description of the Related Art

Morbid obesity is a serious medical condition. In fact, morbid obesity has become highly pervasive in the United States, as well as other countries, and the trend appears to be heading in a negative direction. Complications associated with morbid obesity include hypertension, diabetes, coronary artery disease, stroke, congestive heart failure, multiple orthopedic problems and pulmonary insufficiency with markedly decreased life expectancy. With this in mind, and as those skilled in the art will certainly appreciate, the monetary and physical costs associated with morbid obesity are substantial. In fact, it is estimated the costs relating to obesity are in excess of one hundred billion dollars in the United States alone,

A variety of surgical procedures have been developed to treat obesity. The most common currently performed procedure is Roux-en-Y gastric bypass (RYGB). This procedure is highly complex and is commonly utilized to treat people exhibiting morbid obesity Other forms of bariatric surgery include Fobi pouch, bilio-pancreatic diversion, and gastroplastic or "stomach stapling". In addition, implantable devices are known which limit the passage of food through the stomach and affect satiety.

In view of the highly invasive nature of many of these procedures, efforts have been made to develop less traumatic and less invasive procedures. Gastric-banding is one of these methods. Gastric-banding is a type of gastric reduction surgery attempting to limit food intake by reducing the size of the stomach. In contrast to RYGB and other stomach reduction procedures, gastric-banding does not require the alteration of the anatomy of the digestive tract in the duodenum or jejunum.

Since the early 1980's, gastric bands have provided an effective alternative to gastric bypass and other irreversible surgical weight loss treatments for the morbidly obese. Several alternate procedures are performed under the heading of gastric-banding. Some banding techniques employ a gastric ring, others use a band, some use stomach staples and still other procedures use a combination of rings, bands and staples. Among the procedures most commonly performed are vertical banded gastroplasty (VBG), silastic ring gastroplasty (SRG) and adjustable silastic gastric banding (AGB).

In general, the gastric band is wrapped around an upper portion of the patient's stomach, forming a stoma that is less than the normal interior diameter of the stomach. This restricts food passing from an upper portion to a lower digestive portion of the stomach. When the stoma is of an appropriate size, food held in the upper portion of the stomach provides a feeling of fullness that discourages overeating. Each of patent applications US2005119614, US2007016229, US2005228415, WO2005072195, WO2007080334, and WO2008109300 disclose a gastric band. WO0185071 discloses a gastric band according to the preamble of appended claim 1.

More particularly, and in practice, the gastric band is inserted behind the stomach and the ends of the gastric band are coupled to latch the device about the stomach. However, it is often difficult to manipulate the gastric band during application. As such, the present gastric band has been developed in an effort to alleviate these problems.

### SUMMARY OF THE INVENTION

The present invention provides a gastric band as defined in independent claim 1.

Preferred embodiments are defined in the dependent claims.

### BRIEF DESCRIPTION OF THE DRAWINGS

Figure 1 shows a gastric band in accordance with a first embodiment of the present invention positioned around a stomach.
Figures 2 and 3 respectively show a detailed perspective view and a cross sectional view of the first end of the gastric band shown in Figure 1.

### DESCRIPTION OF THE PREFERRED EMBODIMENTS

The detailed embodiments of the present invention are disclosed herein. It should be understood, however, that the disclosed embodiments are merely exemplary of the invention, which may be embodied in various forms. Therefore, the details disclosed herein are not to be interpreted as limiting, but merely as the basis for teaching one skilled in the art how to make and/or use the invention.

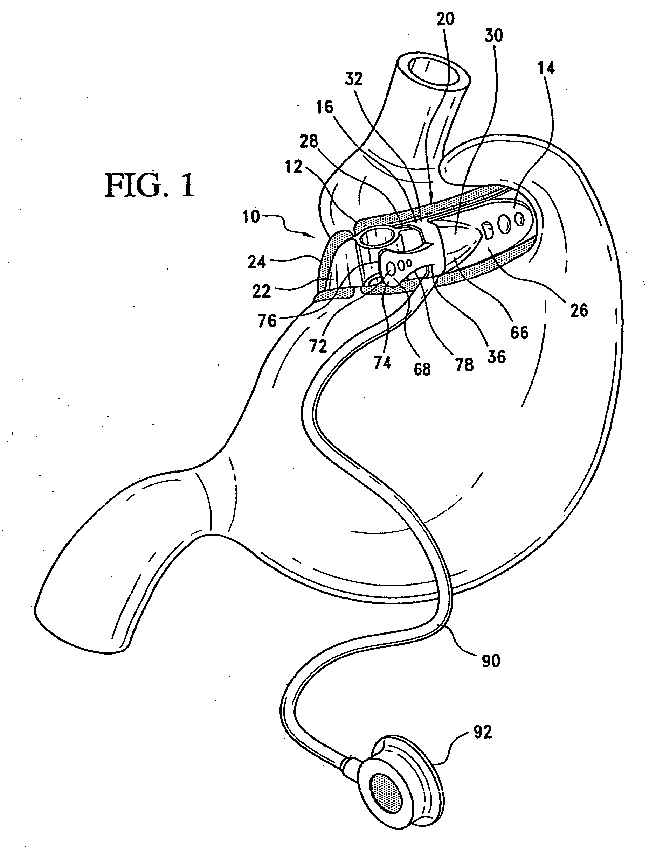

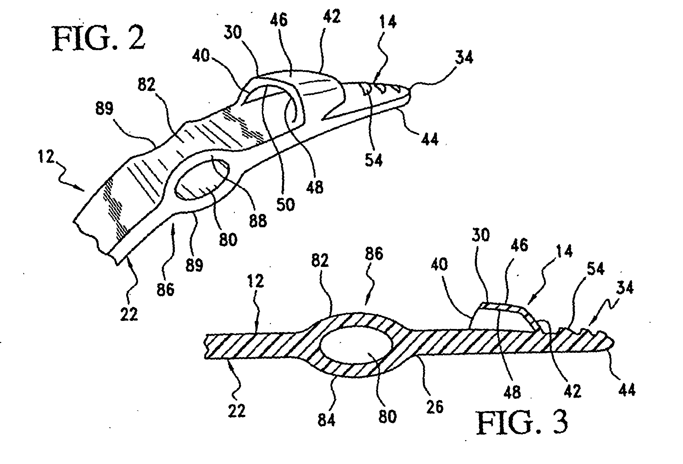

With reference to Figures 1, 2 and 3, a gastric band 10 in accordance with a preferred embodiment of the present invention includes a band body 12 having a first end 14 and an opposite second end 16. A latching mechanism 20 composed of a shell or a first latching member 30 and a collar or a second latching member 32 is secured to the respective ends of the band body 12 in a manner permitting selective attachment of the gastric band 10 about a patient's stomach. The band body 12 and latching mechanism 20 are preferably manufactured from silicone. Although, and as will be discussed below in greater detail, the gastric band 10 is a balloon-type gastric band, the present latching mechanism 20 may be used in conjunction with a variety of band structures without departing from the present invention.

The gastric band 10 is shaped and dimensioned to circumscribe the stomach at a predetermined location reducing the size of the stomach. The gastric band 10 employs a flexible latching mechanism 20 capable of locking and unlocking without destruction of the latching mechanism 20 or significant reduction in retention capabilities after re-locking. The first and second ends 14, 16 respectively include a shell member 30 and a collar member 32 which act as both male and female members depending on the direction of motion and intent to lock or unlock the latching mechanism 20 of the present gastric band 10.

Referring to Figures 1 to 3, and in accordance with a preferred embodiment of the present invention, the gastric band 10, in particular, the gastric band body 12, is generally composed of a reinforcing belt 22 to which an elongated balloon 24 is secured. The belt 22 includes a first end 26 and a second end 28, which generally correspond to the first end 14 and the second end 16 of the gastric band body 12, to which shell member 30 and collar member 32 of the latching mechanism 20 are respectively secured. In accordance with a preferred embodiment, the latching members are the same as disclosed in commonly owned U.S Patent Application Serial No. 11/182,072, entitled "Latching Device for Gastric Band", filed July 15, 2005.

As those skilled in the art will certainly appreciate, the belt 22 includes an inner surface and an outer surface. The outer surface is substantially smooth and forms a substantial portion of the outer surface of the gastric band when it is secured about a patient's stomach. The inner surface of the belt 22 is shaped and dimensioned for attachment to the outer surface of the balloon 24.

With regard to the balloon 24, it also includes a first end, a second end, an inner surface and an outer surface. The inner surface is substantially smooth and is shaped and dimensioned for engaging the patient's stomach when the gastric band is secured thereto. The outer surface of the balloon 24 is shaped and dimensioned for coupling with the inner surface of the belt.

In accordance with a preferred embodiment, the basic construction of the balloon and belt is substantially as disclosed in commonly owned U.S Patent Application Serial Nos. 11/364,362, entitled "Gastric Band", filed March 1, 2006, and 11/364,363, entitled "Precurved Gastric Band", filed March 1, 2006.

As briefly mentioned above, the first end 14 of the gastric band 10 includes the shell member 30. The shell member 30 is generally composed of a hollow, half-moon shaped shell with a tab 34 for gripping and pulling through a collar member 32 composed of a semi-circular shaped aperture 36 on the second end 16 of the gastric band 10. The half-moon shaped shell of the shell member 30 collapses as it is pulled or pushed through the collar member 32 by a grasper. The collar member 32 includes a tongue such that the shell member 30 slides through the semi-circular shaped aperture 36 and under the tongue (not shown) during latching. Once the shell member 30 surface 48 of the shell member 30 adjacent the wide end 40 similarly has a substantially smooth semi-circular profile.

The shell member 30 is compressed and slid through the collar member 32 as discussed above. Thereafter, the center 50 of the wide end 40 returns to its original shape and fits over the tongue. When the gastric band 10 is unlatched, the shell member 30 is pulled forward away from the collar member 32 and the shell member 30 permits it to move under the tongue and through the collar member 32. The preformed shape of the shell member 30 not only acts as a guiding feature for the tongue to slide over the shell member 30 during unlocking, but will also allow the shell member 30 to more easily slide back through the aperture 36 of the collar member 32.

The tab 34 is formed with protrusions 54 assisting in grabbing the tab 34 during locking and unlocking.

Secure fastening of the shell member 30 with the collar member 32 is achieved by ensuring that after the shell member 30 compresses while passing through the collar member 32, the shell member 30 returns to its original shape and the wide end 40 of the shell member 30 abuts with the first edge 66 of the collar member 32.

Latching is further enhanced by providing the collar member 32 with a tongue extending from the collar member 32 away from the tip of the second end 16. The tongue is shaped and dimensioned to seat within the wide end 40 of the shell member 30 after the shell member 30 has passed through the collar member 32 and the gastric band 10 is tensioned as the first and second ends 14, 16 are drawn toward each other with the shell member 30 straining to move back through the collar member 32 toward an unlatched positioned. With this in mind, the tongue may be downwardly oriented such that it slides with the shell member 30 in a convenient and reliable manner. The tongue may be distinctly colored to provided an indication as to whether the latching mechanism 20 is properly locked.

Gripping of the second end 16 is further enhanced through the provision of a forward facing gripping member 68, that is, a gripping member facing the tip of the second end 16. The forward facing gripping member 68 is shaped and dimensioned to permit dual directional access for locking and unlocking of the latching mechanism 20. More particularly, the gripping member 68 includes protrusions 72 along the top and bottom surfaces 74, 76 thereof. These protrusions 72 facilitate gripping thereof along a first directional orientation. The gripping member 68 is further formed with an "hourglass" shape having a reinforced central section 78. The reinforced central section 78 allows for gripping in a second directional orientation.

The gripping member 68 is shaped and dimensioned to receive and center the shell member 30 as it passes through the collar member 32. The gripping member 68 also assists in compressing the shell member 30 as it passes through the collar member 32.

One of the objects of the present gastric band is an improvement to the gastric band's ease of use. With this in mind, various embodiments including an engagement member have been developed facilitating improved access to the first end of the gastric band for grasping during application and removal.

In particular, and with reference to Figures 1, 2 and 3, a gastric band 10 according to the present invention is disclosed wherein an engagement member in the form of a thru-hole 80 is provided in the belt 22 just proximal of the shell member 30 at the first end 14 of the gastric band 10, in particular, at the first end 26 of the belt 22 on the side of the shell member 30 opposite the tip 44 of the first end 14 of the gastric band 10. The thru-hole 80 passes laterally through the belt 22 adjacent the first end 26 thereof. As such, the thru-hole 80 includes a longitudinal axis that is substantially perpendicular to the longitudinal axis of the gastric band 10 when stretched in a substantially flat orientation.

The thru-hole 80 is shaped and dimensioned to allow a surgeon to slide a grasper in and manipulate the gastric band 10 from the side. In addition, the belt 22 region above the thru-hole 80 is tapered inward so that the surgeon can grasp the gastric band 10 from straight above. More particularly, the thru-hole 80 is defined by a top surface 82 and a bottom surface 84, and the top surface 82 is narrower in its lateral dimension (that is, as it extends from a first edge 88 of the belt 22 to the second edge 89 of the belt 22) than the bottom surface 84. As a result, if the surgeon comes straight onto this thru-hole region 86 of the belt 22 with a grasper, the grasper will bottom out on the belt 22 at the bottom surface 84, thereby protecting the balloon 24.

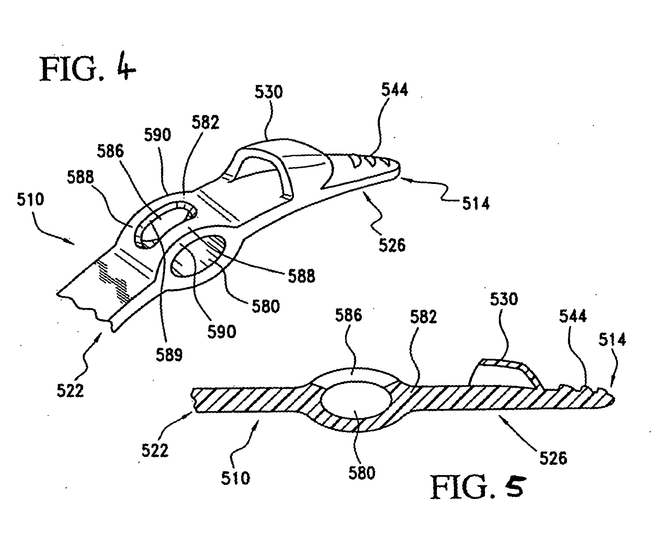

Another embodiment of the present invention is disclosed with reference to Figures 4 and 5. This embodiment employs an engagement member in the form of a thru-hole 580 similar to that described above with reference to Figures 1, 2 and 3. The thru-hole 580 laterally passes through the side of the belt 522 of the gastric band 510 just proximal of the shell member 530, in particular, at the first end 526 of the belt 522 on the side of the shell member 530 opposite the tip 544 of the first end 514 of the gastric band 510. As with the prior embodiment, the thru-hole 580 allows the surgeon to slide a grasper in and manipulate the gastric band 510 from the side. In addition to the side thru-hole 580, a center hole 586 is provided along the top surface 582 of the belt 522 and in alignment with the thru-hole 580. The center hole 586 is positioned to allow access from the top of the gastric band 510. By the inclusion of the center hole 586, a surgeon may grasp either of the thin sections 588 defined by the side walls 589 of the center hole 586 and the edges 590 of the belt 522, aiding in manipulation thereof. The thru-hole 580 and center hole 586 also collapse if the surgeon grasps the entire region. Since there is belt 522 material below the thru-hole 580, that is, along the bottom surface 584 of the belt 522, the balloon 524 is protected in accordance with this concept.

Improved attachment of the present gastric band about a patient's stomach may be achieved through the implementation of a suture tab extender as disclosed in commonly owned U.S. Patent Application Serial No. 11/364,361, entitled "Gastric Band Suture Tab Extender", filed March 1, 2006.

As those skilled in the art will certainly appreciate, a supply tube 90 is used to connect the internal cavity of the balloon 24 of the gastric band 10 with a pressurized fluid source 92, for example, a velocity port. The utilization of the supply tube 90 with a remote fluid source 92 allows for controlled inflation and deflation of the balloon 24 in a predetermined manner. The exact position of the supply tube 90 is important in that the surgeon does not want the supply tube 90 to be a visual obstruction during locking and/or other manipulation of the gastric band 10. In addition, once placement of the gastric band 10 is complete, the supply tube 90 should not cause irritation to surrounding tissue (for example, sticking directly into the liver or spleen). Surgeons also do not want to pull the supply tube 90 through a retro-gastric tunnel, since they cannot easily see if the tissue is being damaged. The supply tube 90 should also be able to act as a safe grasping location for manipulation of the gastric band 10, the supply tube 90 must not kink at the junction to the gastric band 10 and prevent fluid flow, and the supply tube 90 location should facilitate passage of the gastric band 10 through a small trocar.

In accordance with various preferred embodiments of the present invention, different tube placements may be employed as disclosed in commonly owned U.S. Patent Application Serial Nos. 11/364,362, entitled "Gastric Band", filed March 1, 2006, and 11/364,363, entitled "Precurved Gastric Band", filed March 1, 2006.

Although the present invention is described for use in conjunction with gastric bands, those skilled in the art will appreciate the above invention has equal applicability to other types of implantable bands. For example, bands are used for the treatment of fecal incontinence. One such band is described in U.S. Patent No. 6,461,292. Bands can also be used to treat urinary incontinence. One such band is described in U.S. Patent Application Publication No. 2003/0105385. Bands can also be used to treat heartburn and/or acid reflux. One such band is described in U.S. Patent No. 6,470,892. Bands can also be used to treat impotence. One such band is described in U.S. Patent Application Publication No. 2003/0114729.

While the preferred embodiments have been shown and described, it will be understood that there is no intent to limit the invention by such disclosure, but rather, is intended to cover all modifications and alternate constructions falling within the scope of the invention.

## Claims

1. A gastric band (10), comprising:
a gastric band body (12) having a first end (14) and second end (16), the first end and the second end being provided with a respective first latching member (30) and a second latching member (32), the first and second latching members being operable to lock together and unlock to selectively attach the first end of the gastric band to the second end of the gastric band, the first end including a tip (34) and an engagement member (80, 580) proximal the first latching member such that the engagement member is on a side of the first latching member opposite the tip, the engagement member being engageable by a grasper to facilitate a surgeon to manipulate the gastric band during application and/or removal of the gastric band,
**characterized in that** the engagement member is a lateral thru-hole (80, 580) formed in the gastric band body such that the thru-hole has a longitudinal axis that is substantially perpendicular to a longitudinal axis of the gastric band when stretched in a substantially flat orientation, wherein the thruhole (80, 580) includes a top surface (82, 582) and a bottom surface (84, 584), wherein the bottom surface is more adjacent a body lumen when the gastric band is wrapped around the body lumen than the top surface.

2. The gastric band according to claim 1, wherein the top surface (82) is narrower in its lateral dimension than the bottom surface (84).

3. The gastric band according to claim 1, wherein the thru-hole (580) has a center hole (586) extending through the top surface (582) thereof.

## Patentansprüche

1. Magenband (10), das Folgendes umfasst:
einen Magenbandkörper (12) mit einem ersten Ende (14) und einem zweiten Ende (16), wobei das erste Ende und das zweite Ende mit einem ersten Arretierungsglied (30) bzw. einem zweiten Arretierungsglied (32) versehen sind, wobei die ersten und zweiten Arretierungsglieder verriegelt und voneinander gelöst werden können, um das erste Ende des Magenbands selektiv am zweiten Ende des Magenbands zu befestigen, wobei das erste Ende eine Spitze (34) und ein Eingriffsglied (80, 580) proximal zum ersten Arretierungsglied aufweist, so dass sich das Eingriffsglied auf einer Seite des ersten Arretierungsglieds gegenüber der Spitze befindet, wobei das Eingriffsglied von einem Greifer ergriffen werden kann, um einem Chirurgen die Manipulation des Magenbands während der Applikation und/oder Entfernung des Magenbands zu erleichtern,
**dadurch gekennzeichnet, dass** das Eingriffsglied eine seitliches Durchgangsloch (80, 580) im Magenbandkörper ist, so dass das Durchgangsloch eine Längsachse aufweist, die im Wesentlichen senkrecht zu einer Längsachse des Magenbands verläuft, wenn dieses in einer im Wesentlichen flachen Orientierung gedehnt wird, worin das Durchgangsloch (80, 580) eine Oberseite (82, 582) und eine Unterseite (84, 584) aufweist, worin die Unterseite näher am Körperlumen liegt, wenn das Magenband um das Körperlumen gewickelt wird, als die Oberseite.

2. Magenband nach Anspruch 1, worin die Oberseite (82) in ihrer seitlichen Abmessung schmäler ist als die Unterseite (84).

3. Magenband nach Anspruch 1, worin das Durchgangsloch (580) ein Mittelloch (586) aufweist, das sich durch seine Oberseite (582) erstreckt.

## Revendications

1. Bande (10) gastrique, comportant :
un corps (12) de bande gastrique présentant une première extrémité (14) et une seconde extrémité (16), la première extrémité et la seconde extrémité étant pourvues respectivement d'un premier élément (30) de verrouillage et d'un second élément (32) de verrouillage, les premier et second éléments de verrouillage servant à se verrouiller ensemble et à se déverrouiller pour attacher de manière sélective la première extrémité de la bande gastrique à la seconde extrémité de la bande gastrique, la première extrémité comprenant une pointe (34) et un élément (80, 580) d'engagement proximal par rapport au premier élément de verrouillage de sorte que l'élément d'engagement est situé sur un côté du premier élément de verrouillage à l'opposé de la pointe, l'élément d'engagement pouvant être engagé par une pince pour faciliter la manipulation de la bande gastrique par un chirurgien lors de l'application et/ou du retrait de la bande gastrique,
**caractérisée en ce que** l'élément d'engagement est un trou traversant (80, 580) latéral formé dans le corps de la bande gastrique de sorte que le trou traversant possède un axe longitudinal qui est sensiblement perpendiculaire à un axe longitudinal de la bande gastrique lorsqu'étiré dans une orientation sensiblement plane, le trou traversant (80, 580) comprenant une surface (82, 582) supérieure et une surface (84, 584) inférieure, la surface inférieure étant en position plus adjacente à une lumière corporelle lorsque la bande gastrique est enroulée autour de la lumière corporelle que la surface supérieure.

2. Bande gastrique selon la revendication 1, dans laquelle la surface (82) supérieure est plus étroite dans sa dimension latérale que la surface (84) inférieure.

3. Bande gastrique selon la revendication 1, dans laquelle le trou traversant (580) présente un trou (586) central traversant la surface (582) supérieure de celle-ci.
